(19) Europäisches Patentamt
European Patent Office
Office européen des brevets

(11) **EP 4 040 151 B1**

(12) **EUROPEAN PATENT SPECIFICATION**

(45) Date of publication and mention
of the grant of the patent:
**13.08.2025 Bulletin 2025/33**

(21) Application number: **20871761.1**

(22) Date of filing: **01.09.2020**

(51) International Patent Classification (IPC):
**G01N 33/48** (2006.01)   **G01N 33/543** (2006.01)
**G01N 33/53** (2006.01)

(52) Cooperative Patent Classification (CPC):
**G01N 33/62; B01L 3/0289; G01N 1/405;
G01N 33/5302; G01N 33/5306; G01N 33/54388;
G01N 33/54393;** B01L 2200/0631; B01L 2300/028;
B01L 2300/0832; B01L 2300/0854; B01L 2400/0478

(86) International application number:
**PCT/JP2020/033152**

(87) International publication number:
**WO 2021/065300 (08.04.2021 Gazette 2021/14)**

(54) **IMMUNOLOGICAL TEST METHOD**

IMMUNOLOGISCHES PRÜFVERFAHREN

MÉTHODE D'ESSAI IMMUNOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2019 JP 2019178682
16.01.2020 JP 2020005325
03.06.2020 JP 2020096898**

(43) Date of publication of application:
**10.08.2022 Bulletin 2022/32**

(73) Proprietor: **FUJIFILM Corporation
Tokyo 106-8620 (JP)**

(72) Inventors:
• **ABURAYA, Yoshihiro
Ashigarakami-gun, Kanagawa 258-8538 (JP)**
• **NISHIKAWA, Naoyuki
Ashigarakami-gun, Kanagawa 258-8538 (JP)**
• **KATADA, Junichi
Ashigarakami-gun, Kanagawa 258-8538 (JP)**
• **WADA, Atsuhiko
Ashigarakami-gun, Kanagawa 258-8538 (JP)**

• **UJIHARA, Dai
Ashigarakami-gun, Kanagawa 258-8538 (JP)**

(74) Representative: **Dehns Germany Partnerschaft
mbB
Theresienstraße 6-8
80333 München (DE)**

(56) References cited:
**EP-A1- 1 234 165          WO-A1-01/40762
WO-A1-2005/058453     WO-A1-2006/007711
CN-A- 109 569 023        JP-A- 2013 543 110
JP-B2- 5 728 453           US-A1- 2013 231 460
US-A1- 2014 087 367**

• **XIE XING ET AL: ""Nanofiltration" Enabled by
Super-Absorbent Polymer Beads for
Concentrating Microorganisms in Water
Samples", SCIENTIFIC REPORTS, vol. 6, no. 1,
15 February 2016 (2016-02-15), XP055968822,
Retrieved from the Internet <URL:http://www.
nature.com/articles/srep20516.pdf> DOI:
10.1038/srep20516**

**Description**

**BACKGROUND OF THE INVENTION**

1. Field of the Invention

The present invention relates to an immunological test method.

2. Description of the Related Art

Immunological test methods (particularly, immunochromatography) are frequently used these days since the operation is easy and measurement can be carried out in a short time.

For example, in a case where an antigen such as influenza virus is detected by immunochromatography, the following operation is carried out.

First, a label modified with an antibody (labeled antibody) is prepared and mixed with a specimen containing an antigen. The labeled antibody binds to an antigen, whereby composite bodies are formed. In this state, in a case where these composite bodies are spread on an insoluble carrier having a detection line (a test line) onto which an antibody that specifically reacts with an antigen is applied, the composite bodies react with the antibody on the detection line and are captured, and detection is confirmed visually or in other manners.

Examples of such immunochromatography include the immunochromatography disclosed in JP5728453B.

WO 2006/007711 A1 discloses a sample applicator for transferring a fluid sample including a syringe body defining a hollow bore with an open end and an outlet, a plunger having a piston at one end and being effective to fit within the hollow bore, and a second end extending out of the open end when the piston is located within the hollow bore. The syringe applicator further includes at least one of a reagent and a concentrating material and a fluid dye material, located between the piston and the outlet.

CN 109 569 023 A discloses decontaminant-doped dry hydrogel particles and a method for realizing mass concentration of a macromolecular liquid sample and specific activity improvement of a protein liquid sample by utilizing the decontaminant-doped dry hydrogel particles.

US 2013/231460 A1 discloses method for concentrating one or more target compounds in a liquid sample, a device for carrying out this method and a kit for processing a biological sample comprising such a device.

XIE XING ET AL: ""Nanofiltration" Enabled by Super-Absorbent Polymer Beads for Concentrating Microorganisms in Water Samples", SCIENTIFIC REPORTS, vol. 6, no. 1, 15 February 2016 discloses detection and quantification of pathogens in water is critical for the protection of human health and for drinking water safety and security using superabsorbent polymer (SAP) beads.

US 2014/087367 A1 discloses a chromatography method including a step of forming a composite with a test substance and a labeling substance containing a metal modified by a first binding substance of the test substance and then developing the composite on an insoluble carrier; a step of capturing the test substance and the labeling substance in a detection site on the insoluble carrier including a second binding substance of the test substance or a substance having a binding property to the first binding substance of the test substance; and a step of amplifying the captured labeling substance using a first amplification reagent and a second amplification reagent to detect the test substance.

EP 1 234 165 A1 discloses a sample container which contains a hygroscopic substance and which makes it possible to directly conserve samples and/or assign material containing DNA/RNA at the location of extraction. The invention also relates to the use of said container for storing/preserving samples that contain DNA/RNA.

**SUMMARY OF THE INVENTION**

These days, there is a demand for an immunodiagnostic method that can be applied to a sample solution having an extremely low antigen concentration. For this reason, regarding immunological test methods such as immunochromatography, there is also a demand for a method having higher sensitivity than the method in the related art, as disclosed in JP5728453B.

In consideration of the above circumstances, an object of the present invention is to provide an immunological test method having high detection sensitivity. Against this background, the invention proposes an immunological test method according to claim 1. Preferred embodiments are the subject of the dependent claims as well as the following description.

As a result of intensive studies with regard to the above object, inventors of the present invention have found that the above object can be achieved by using a sample solution concentrated according to a predetermined method and have reached the present invention.

That is, the inventors of the present invention have found that the object can be achieved by the following configurations.

(1) An immunological test method comprising:

a concentration step of concentrating an antigen-containable solution by mixing the antigen-containable solution with a superabsorbent polymer to obtain an antigen-concentrated solution by incorporating impurities including urea contained in the antigen-containable solution together with water into the superabsorbent polymer; and a detection step of detecting an antigen in the antigen-concentrated solution using an antigen-antibody reaction, in which the swelling ratio of the superabsorbent polymer ranges from including 20 g/g to including 500 g/g, wherein the swelling ration is defined as grams of water retained by one gram of the superabsorbent polymer, and an antibody that is used in the antigen-antibody reaction is a monoclonal antibody, wherein the superabsorbent polymer is a polyacrylic acid-based, polyacrylamide-based, cellulose-based, or polyethylene oxide-based polymer, wherein the antigen-containable solution is urine, wherein a concentration of urea in the antigen-concentrated solution is 5 times or less with respect to a concentration of urea in the antigen-containable solution.

(2) The immunological test method according to (1), in which a particle diameter of the superabsorbent polymer is 5 mm or less.
(3) The immunological test method according to (1) or (2), in which a water absorption rate of the superabsorbent polymer is 0.01 g/min or more and 40 g/min or less per 1 g of the superabsorbent polymer.
(4) The immunological test method according to any one of (1) to (3), in which the immunological test method is immunochromatography.
(5) The immunological test method according to (4), in which the detection step includes:

a spreading step of spreading gold particle composite bodies on an insoluble carrier having a reaction site at which a second binding substance capable of binding to an antigen in the antigen-concentrated solution has been immobilized, in a state where the gold particle composite bodies which are composite bodies of the antigen and modified gold particles which are gold particles modified with a first binding substance capable of binding to the antigen are formed, a capturing step of capturing the gold particle composite bodies at the reaction site of the insoluble carrier, and a silver amplification step of silver-amplifying the gold particle composite body captured in the capturing step.

(6) The immunological test method according to any one of (1) to (5), wherein in the concentration step a concentration jig that is used that comprises:

a container that accommodates the superabsorbent polymer, in which the container has an incorporation part for incorporating the antigen-containable solution and a discharge unit for discharging the antigen-concentrated solution.

(7) The immunological test method according to (6), wherein the concentration jig comprises:

a cylinder that is the container; and a piston that is insertable into the cylinder.

(8) The immunological test method according to (6), wherein the concentration jig comprises:

an inner tube that is the container; and an outer tube into which the inner tube is insertable and from which the inner tube is removable.

(9) The immunological test method according to (6), wherein the concentration jig, wherein the concentration jig has a dropper shape having a tube part and a pump part, and the tube part is the container.

As shown below, according to the present invention, it is possible to provide an immunological test method that has a high detection sensitivity and a concentration jig that is used for this immunological test method. The concentration jig is useful for practising embodiments of the invention, but is not claimed as such.

## BRIEF DESCRIPTION OF THE DRAWINGS

Fig. 1 is a schematic view illustrating an aspect of an insoluble carrier that is used in a method according to the embodiment of the present invention.
Fig. 2 is a schematic cross-sectional view illustrating an aspect of a suitable aspect 1 of a concentration jig used in a concentration step according to the embodiment of the present invention.
Fig. 3 is a schematic cross-sectional view illustrating an aspect of a suitable aspect 2 of the concentration jig used in a concentration step according to the embodiment of the present invention.
Fig. 4 is a schematic cross-sectional view illustrating an aspect of a suitable aspect 3 of the concentration jig used in a concentration step according to the embodiment of the present invention.

## DESCRIPTION OF THE PREFERRED EMBODIMENTS

Hereinafter, an immunological test method according to the embodiment of the present invention and a concentration jig that is used in the immunological test method according to the embodiment of the present invention will be described. Only methods are claimed. The jig is useful for practising embodiments of the invention but is not claimed as such.

In the present specification, the numerical value range indicated by using "to" means a range including the numerical values before and after "to" as the lower limit value and the upper limit value, respectively.

In addition, in the present specification, one kind of each component may be used alone, or two or more kinds thereof may be used in combination. In a case where two or more kinds of each component are used in combination, a content of the component indicates a total content unless otherwise specified.

Further, in the present specification, "the detection sensitivity and the signal/noise ratio (the S/N ratio) are further improved" is also described as "the effects and the like of the present invention are more excellent".

Immunological test method

The immunological test method according to the embodiment of the present invention (hereinafter, also referred to as "the method according to the embodiment of the present invention") is an immunological test method including;

a concentration step of concentrating an antigen-containable solution by mixing the antigen-containable solution with a superabsorbent polymer to obtain an antigen-concentrated solution by incorporating impurities including urea contained in the antigen-containable solution together with water into the superabsorbent polymer; and
a detection step of detecting an antigen in the antigen-concentrated solution using an antigen-antibody reaction,
in which the swelling ratio of the superabsorbent polymer ranges from including 20 g/g to including 500 g/g, wherein the swelling ration is defined as grams of water retained by one gram of the superabsorbent polymer, and
an antibody that is used in the antigen-antibody reaction is a monoclonal antibody,
wherein the superabsorbent polymer is a polyacrylic acid-based, polyacrylamide-based, cellulose-based, or polyethylene oxide-based polymer,
wherein the antigen-containable solution is urine,
wherein a concentration of urea in the antigen-concentrated solution is 5 times or less with respect to a concentration of urea in the antigen-containable solution.

It is presumed that since the method according to the embodiment of the present invention has such a configuration, the above effects can be obtained. The reason for this is not clear; however, it is conceived to be as follows.

As described above, in the method according to the embodiment of the present invention, an antigen-containable solution (a sample solution) is concentrated using a superabsorbent polymer having a specific swelling ratio. In a case where the sample solution and the superabsorbent polymer are mixed, the water in the sample solution is incorporated into the superabsorbent polymer, whereas an antigen in the sample solution is hardly incorporated into the superabsorbent polymer since the antigen has a certain degree of hydrodynamic radius and thus the network structure on the surface of the superabsorbent polymer exhibits a sieving effect. As a result, the antigen in the sample solution is concentrated, which leads to the improvement in detection sensitivity.

On the other hand, the sample solution generally contains impurities such as low molecular weight components and salts. For example, in a case where the sample solution is urine, it contains impurities such as urea. From the studies by inventors of the present invention, it was found that in a case where these impurities are concentrated together with an antigen, the antigen-antibody reaction is inhibited and the detection sensitivity is decreased. That is, it is known that the effect of improving the detection sensitivity by concentration cannot be sufficiently obtained.

The method according to the embodiment of the present invention is based on the above findings. That is, in the method according to the embodiment of the present invention, these impurities are incorporated into the superabsorbent polymer together with water since a superabsorbent polymer having a specific swelling ratio is used as the superabsorbent polymer. For this reason, the above-described decrease in detection sensitivity hardly occurs. As a result, it is conceived that an extremely high detection sensitivity is achieved.

Hereinafter, each of the steps included in the method according to the embodiment of the present invention will be described.

[Concentration step]

The concentration step is a step of concentrating an antigen-containable solution (a sample solution) by mixing the antigen-containable solution with a superabsorbent polymer to obtain an antigen-concentrated solution (a solution in which an antigen is concentrated). Here, the swelling ratio of the superabsorbent polymer ranges from including 20 g/g to including 500 g/g.

[Sample solution]

The sample solution that is used in the concentration step is not particularly limited as long as it is an antigen-containable solution including urea.

The sample solution is urine due to the reason that the effects of the present invention are more excellent.

As the sample solution is urine, the concentration of urea in the antigen-concentrated solution obtained in the concentration step is 5 times or less with respect to the concentration of urea in the sample solution due to the reason that the effects and the like of the present invention are more excellent.

<Antigen>

Examples of the antigen include a fungus, a bacterium (for example, tubercle bacillus or lipoarabinomannan (LAM) included in the tubercle bacillus), a virus (for example, an influenza virus), and a nuclear protein thereof. LAM is a major antigen in tuberculosis and a glycolipid which is a major constitutional component of the cell membrane and the cell wall.

The antigen is more preferably a virus (particularly, an influenza virus) or LAM and still more preferably LAM due to the reason that the effects and the like of the present invention are more excellent.

<Pretreatment of sample solution>

Regarding the sample solution, it is possible to use a sample solution as it is or in a form of an extraction solution obtained by extracting an antigen using an appropriate solvent for extraction, in a form of a diluent solution obtained by diluting an extraction solution with an appropriate diluent, or in a form in which an extraction solution has been concentrated by an appropriate method.

As the solvent for extraction, it is possible to use a solvent (for example, water, physiological saline, and a buffer solution) that is used in a general immunological analysis method, or a water-miscible organic solvent with which a direct antigen-antibody reaction can be carried out by being diluted with such a solvent.

[superabsorbent polymer]

The superabsorbent polymer that is used in the concentration step is a polymer having a swelling ratio that ranges from including 20. g/g to including 500 g/g (hereinafter, also referred to as a "specific superabsorbent polymer"). Here, the swelling ratio is a value defined as "a mass (g) of water retained by 1 g of a superabsorbent polymer".

The specific superabsorbent polymer is not particularly limited as long as the swelling ratio thereof ranges from including 20 g/g to including 500 g/g; however, due to the reason that the effects and the like of the present invention are more excellent, the superabsorbent polymer is a polyacrylic acid-based, polyacrylamide-based, cellulose-based, or polyethylene oxide-based polymer.

<Swelling ratio>

As described above, the swelling ratio of the specific superabsorbent polymer ranges from including 20 g/g to including 500 g/g. Among the above, due to the reason that the effects and the like of the present invention are more excellent, it is preferably 100 g/g or less.

(Method of measuring swelling ratio)

A mass of a superabsorbent polymer stored for 10 days at 25°C and 5% of relative humidity (RH) is measured, and immediately after the measurement, the superabsorbent polymer is immersed in a large amount of distilled water. After 120 minutes, the superabsorbent polymer is taken out, the water on the surface thereof is removed, the mass thereof is measured again, and the swelling ratio thereof is measured using the following expression.

{(Mass (g) after water absorption -initial mass (g) before water absorption)/initial mass (g) before water absorption}

The method of adjusting the swelling ratio to the above-described specific range is not particularly limited; however, examples thereof include changing the kind of the polymer, changing the molecular weight of the polymer, changing the degree of crosslinking of the polymer, and changing the particle diameter of the polymer.

<Water absorption rate>

The water absorption rate of the specific superabsorbent polymer is not particularly limited; however, due to the reason that the effects and the like of the present invention are more excellent, it is preferably 0.01 g/min or more and 40 g/min or less per 1 g of superabsorbent polymer, and more preferably 0.02 g/min or more and 40 g/min or less per 1 g of superabsorbent polymer.

The water absorption rate is measured as follows.

A mass (a mass $M_0$, unit: g) of a superabsorbent polymer stored for 10 days at 25°C and 5% of relative humidity (RH) is measured, and immediately after the measurement, the superabsorbent polymer is immersed in a large amount of distilled water. After 10 minutes, the superabsorbent polymer is taken out, the water on the surface thereof is removed, and the mass thereof (a mass $M_{10}$) is measured. Immediately after the mass measurement, the superabsorbent polymer is immersed again in a large amount of distilled water. After 10 minutes, the superabsorbent polymer is taken out, the water on the surface thereof is removed, and the mass thereof (a mass $M_{20}$) is measured again. Immediately after the measurement of mass $M_{20}$, the superabsorbent polymer is immersed again in a large amount of distilled water. After 10 minutes, the superabsorbent polymer is taken out, the water on the surface thereof is removed, and the mass thereof (a mass $M_{30}$) is measured again.

The water absorption amount is defined as follows.

Water absorption amount for 10 minutes: $\Delta M10 = (M_{10} - M_0)/M_0$
Water absorption amount for 20 minutes: $\Delta M20 = (M_{20} - M_0)/M_0$
Water absorption amount for 30 minutes: $\Delta M30 = (M_{30} - M_0)/M_0$

Using the water absorption amount defined as described above, the water absorption rate is calculated as follows.

Three points are plotted on the X-Y plane as the horizontal axis of time (x = 10, 20, 30, unit: minute) and the vertical axis of water absorption amount (y = $\Delta M10$, $\Delta M20$, $\Delta M30$, unit: g (water)/g (polymer amount)), to obtain a linear approximate expression of the water absorption amount with respect to the time by using the least squares method, and the slope of the linear approximate expression is defined as the water absorption rate per unit time (minute).

<Particle diameter>

The specific superabsorbent polymer preferably has a particle shape, and the particle diameter in such a case is preferably 10 mm or less, more preferably 8 mm or less, and still more preferably 5 mm or less, due to the reason that the effects and the like of the present invention are more excellent. The lower limit of the particle diameter of the specific superabsorbent polymer is preferably 0.001 mm or more, more preferably 0.005 mm or more, and still more preferably 0.01 mm or more, due to the reason that the effects and the like of the present invention are more excellent. According to a method of measuring a particle diameter of a specific superabsorbent polymer having a particle shape, the diameters of 50 particles of the polymer having a particle shape are measured with an optical microscope, and the arithmetic mean value thereof can be used as the particle diameter.

<Using amount>

The using amount of the specific superabsorbent polymer is not particularly limited; however, it is preferably 0.01 to 100 g and more preferably 0.1 to 50 g with respect to 1 mL of the sample solution due to the reason that the effects and the like of the present invention are more excellent.

[Procedure of concentration step]

The procedure of the concentration step is not particularly limited; however, examples thereof include a method of mixing the sample solution and the specific superabsorbent polymer to recover a sample solution (an antigen-concentrated solution) that has not been absorbed by the specific superabsorbent polymer.
The method of mixing the sample solution and the specific superabsorbent polymer is not particularly limited, and examples thereof include a method of blending the sample solution with the specific superabsorbent polymer and stirring the blended mixture followed by being allowed to stand.
In the concentration step, it is preferable to use a concentration jig described later due to the reason that the effects and the like of the present invention are more excellent.

[Detection step]

The detection step is a step of detecting an antigen in the antigen-concentrated solution obtained in the concentration step described above, by using an antigen-antibody reaction. Here, the antibody that is used in the antigen-antibody reaction is a monoclonal antibody. Accordingly, there is little concern of false positiveness in the method according to the embodiment of the present invention.
The detection step is not particularly limited as long as an antigen-antibody reaction is used, and examples thereof include an enzyme-linked immuno-sorbent assay (EIA), a solid phase enzyme-linked immuno-sorbent assay (ELISA), a radioimmunoassay (RIA), a fluorescent immunoassay (FIA), a Western blot method, and immunochromatography. Among the above, immunochromatography is preferable due to the reason that the effects and the like of the present invention are more excellent. That is, the method according to the embodiment of the present invention is preferably immunochromatography.
Hereinafter, a suitable aspect in a case where the detection step is immunochromatography will be described.
Due to the reason that the effects and the like of the present invention are more excellent, the detection step preferably includes;

a spreading step of spreading gold particle composite bodies on an insoluble carrier having a reaction site at which a second binding substance capable of binding to an antigen in the antigen-concentrated solution has been immobilized, in a state where the gold particle composite bodies which are composite bodies of the antigen and modified gold particles which are gold particles modified with a first binding substance capable of binding to the antigen are formed,
a capturing step of capturing the gold particle composite bodies at the reaction site of the insoluble carrier, and
a silver amplification step of silver-amplifying the gold particle composite body captured in the capturing step.

Here, at least one of the first binding substance or the second binding substance is a monoclonal antibody. It is preferable that both the first binding substance and the second binding substance are a monoclonal antibody due to the reason that the effects and the like of the present invention are more excellent.
Hereinafter, each of the steps included in the above suitable aspect will be described.

[Spreading step]

The spreading step is a step of spreading gold particle composite bodies on an insoluble carrier having a reaction site at which a second binding substance capable of binding to an antigen in the antigen-concentrated solution obtained in the above-described concentration step has been immobilized, in a state where the gold particle composite body which are composite bodies of the antigen and modified gold particles which are gold particles modified with a first binding substance capable of binding to the antigen are formed.

[Gold particle composite body]

As described above, in the spreading step, first, the gold particle composite body which is a composite body of the antigen in the antigen-concentrated solution obtained in the above-described concentration step and a modified gold particle which is a gold particle modified with a first binding substance capable of binding to the antigen is formed.

<Modified gold particle>

The modified gold particle is a gold particle modified with the first binding substance capable of binding to an antigen.

(Gold particle)

Gold particle is not particularly limited.

The gold particle acts as a catalyst that reduces silver ions in the silver amplification step described later.

The particle diameter of the gold particles is preferably 100 nm or less, more preferably 50 nm or less, still more preferably 30 nm or less, and particularly preferably 15 nm or less, due to the reason that the effects and the like of the present invention are more excellent.

The lower limit of the particle diameter of the gold particles is not particularly limited; however, it is preferably 1 nm or more, more preferably 2 nm or more, and still more preferably 5 nm or more, due to the reason that the effects and the like of the present invention are more excellent.

The particle diameter can be measured with a commercially available particle diameter distribution meter or the like. As a method of measuring the particle size distribution, optical microscopy, confocal laser microscopy, electron microscopy, atomic force microscopy, static light scattering method, laser diffraction method, dynamic light scattering method, centrifugal sedimentation method, electric pulse measurement method, chromatography method, ultrasonic attenuation method, and the like are known, and apparatuses corresponding to the respective principles are commercially available. As the method of measuring a particle diameter, a dynamic light scattering method can be preferably used due to the particle diameter range and the ease of measurement. Examples of the commercially available measuring device using dynamic light scattering include NANOTRAC UPA (Nikkiso Co., Ltd.), a dynamic light scattering type particle size distribution measuring device LB-550 (HORIBA, Ltd.), and a Fiber-Optics Particle Analyzer FPAR-1000 (Otsuka Electronics Co., Ltd.). In the present invention, the average particle size is obtained as a value of a median diameter (d = 50) measured at a measurement temperature of 25°C.

(First binding substance)

The first binding substance is not particularly limited as long as it is capable of binding to the above antigen; however, due to the reason that the effects and the like of the present invention are more excellent, it is preferably a protein, more preferably an antibody (for example, a polyclonal antibody or a monoclonal antibody), and from the viewpoint of achieving higher detection sensitivity, it is still more preferably a monoclonal antibody.

The above antibody is not particularly limited. However, it is possible to use, for example, an antiserum prepared from a serum of an animal immunized with an antigen, or an immunoglobulin fraction purified from an antiserum. In addition, it is possible to use a monoclonal antibody obtained by cell fusion using spleen cells of an animal immunized with an antigen, or a fragment thereof [for example, $F(ab')_2$, Fab, Fab', or Fv]. The preparation of these antibodies can be carried out by a conventional method.

In a case where the antigen is LAM, examples of the first binding substance include the A194-01 antibody described in WO2017/139153A.

In a case where the antigen is LAM, other examples of the first binding substance include the antibody having a sequence described as MoAb1 in paragraph No. [0080] of WO2013/129634A.

(Method of manufacturing modified gold particle)

The method of manufacturing the modified gold particle is not particularly limited, and a known method can be used.

Examples thereof include a chemical bonding method such as a method in which an SH group is introduced into an antibody, and the fact that gold and an SH group are chemically bonded is utilized so that the SH bond of the antibody is cleaved to generate an Au-S bond on the Au surface when the antibody approaches gold particles, whereby the antibody is immobilized.

[Insoluble carrier]

The above-described insoluble carrier is an insoluble carrier having a reaction site (a test line) at which a second binding substance capable of binding to the antigen is immobilized. The insoluble carrier may have a plurality of test lines depending on the kinds of antigens (for example, a test line for influenza A type virus and a test line for influenza B type virus). In addition, the insoluble carrier may have a control line on the downstream side of the test line in order to check the spreading of the gold particle composite bodies. Further, in a case where a reducing agent solution is used in the silver amplification step described later, a coloring reagent immobilization line may be provided downstream of the test line in order to detect the reducing agent solution.

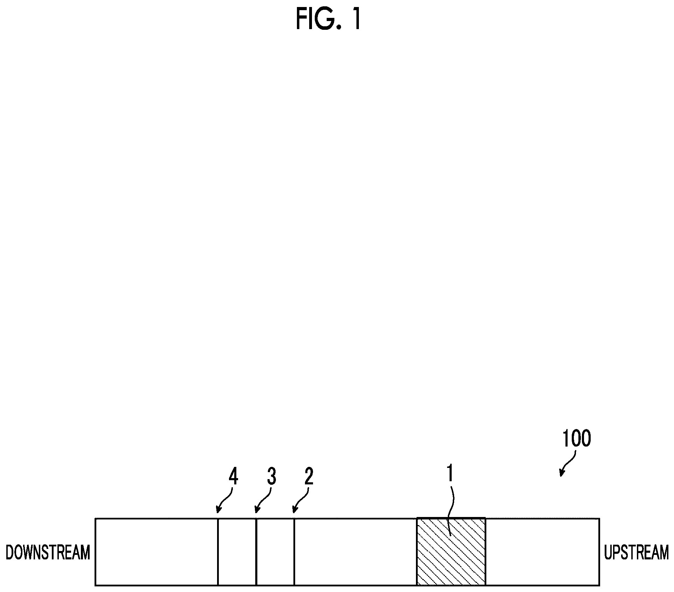

Examples of the specific aspect of the insoluble carrier include a nitrocellulose membrane 100 as illustrated in Fig. 1, which has from the upstream side; a gold colloid holding pad 1, a test line 2, a control line 3, and a coloring reagent immobilization line 4. Here, the gold colloid holding pad 1 is a pad that holds gold particles (modified gold particles) modified with the first binding substance, the test line 2 is a line on which the second binding substance is immobilized, the control line 3 is a line for checking the spreading, and the coloring reagent immobilization line 4 is a line for detecting the reducing agent solution described later. Here, the upstream side and the downstream side mean descriptions intended to indicate the spreading from the upstream side to the downstream side at the time when gold particle composite bodies are spread.

The more specific aspect of the insoluble carrier (or an immunochromatographic kit having the insoluble carrier) include, for example, the insoluble carrier or the immunochromatographic kit disclosed in JP5728453B.

<Insoluble carrier>

The insoluble carrier is preferably a porous carrier. In particular, due to the reason that the effects and the like of the present invention are more excellent, it is preferably a nitrocellulose film (a nitrocellulose membrane), a cellulose membrane, an acetyl cellulose membrane, a polysulfone membrane, a polyether sulfone membrane, a nylon membrane, a glass fiber, a non-woven fabric, a cloth, a thread, or the like is preferable, and a nitrocellulose film is more preferable.

<Second binding substance>

The second binding substance is not particularly limited as long as it is capable of binding to the above antigen.

Specific examples and the suitable aspect of the second binding substance are respectively the same as those of the above-described first binding substance.

The second binding substance may be the same as or different from the above-described first binding substance; however, an aspect in which the second binding substance is a different substance is preferable due to the reason that the effects and the like of the present invention are more excellent.

In addition, in a case where the first binding substance and the second binding substance are antibodies, an aspect in which the antibody which is the first binding substance and the antibody which is the second binding substance are different from each other is preferable due to the reason that the effects and the like of the present invention are more excellent.

Further, in a case where the first binding substance and the second binding substance are antibodies, an aspect in which an epitope (a part of the antigen recognized by the first binding substance) of the first binding substance and an epitope (a part of the antigen recognized by the second binding substance) of the second binding substance are different from each other is preferable due to the reason that the effects and the like of the present invention are more excellent. The difference in epitope between antibodies can be confirmed by, for example, an enzyme-linked immuno-sorbent assay (ELISA).

[Spreading]

The method of spreading gold particle composite bodies on an insoluble carrier having a test line in a state where the gold particle composite bodies are formed is not particularly limited; however, examples thereof include a method in which the above nitrocellulose membrane 100 (or an immunochromatographic kit having the nitrocellulose membrane 100) as illustrated in Fig. 1 is prepared, and the antigen-concentrated solution obtained in the above-described concentration step is dropwise added onto a gold colloid holding pad and moved from the upstream side to the downstream side by using the capillary phenomenon as illustrated in Fig. 1.

[Capturing step]

The capturing step is a step of capturing the gold particle composite bodies at the reaction site of the insoluble carrier.

As described above, since the second binding substance capable of binding to an antigen is immobilized at the reaction site of the insoluble carrier, the gold particle composite bodies (the composite bodies of an antigen and modified gold particles) spread on the insoluble carrier in the spreading step is captured at the reaction site (the test line) of the insoluble carrier.

In a case where a sample solution does not contain an antigen, the above gold particle composite bodies are not formed, and thus the gold particle composite bodies are not captured at the reaction site of the insoluble carrier.

[Silver amplification step]

The silver amplification step is a step of silver-amplifying the gold particle composite body captured in the capturing step.

The silver amplification step is a step of forming large silver particles in the gold particle composite body captured at the reaction site of the insoluble carrier by providing silver ions to the insoluble carrier after the capturing step. More specifically, it is a step in which silver ions are reduced using gold particles of the gold particle composite body as a catalyst to form silver particles (for example, a diameter of 10 $\mu$m or more).

This significantly improves the detection sensitivity of the captured gold particle composite body.

[Suitable aspect]

The method of providing silver ions to the insoluble carrier after the capturing step is not particularly limited; however, it is preferably a method in which the following reducing agent solution and the following silver amplification solution are used, due to the reason that the effects and the like of the present invention are more excellent.

Further, in addition to the reducing agent solution and the silver amplification solution, a washing solution may be used to wash the composite body remaining on the insoluble carrier except for the specific binding reaction. The reducing agent solution may also serve as a washing solution.

<Reducing agent solution>

The reducing agent solution contains a reducing agent capable of reducing silver ions. As the reducing agent capable of reducing silver ions, any inorganic or organic material or a mixture thereof can be used as long as it can reduce silver ions to silver. Preferred examples of the inorganic reducing agent include a reducing metal salt and a reducing metal complex salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by complexing or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), and therefore detoxification is possible. In the present invention, it is preferable to use such an inorganic reducing agent, and as a more preferable aspect of the present invention, it is preferable to use a metal salt of $Fe^{2+}$ as the reducing agent.

It is also possible to use, as the reducing agent, a main developing agent (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), or leuco dyes) that is used in a wet-type light-sensitive silver halide photographic material, and other materials obvious to those who are skilled in the technology in the present field, such as a material disclosed in US6020117A.

As the reducing agent, an ascorbic acid reducing agent is also preferable. The useful ascorbic acid reducing agent includes ascorbic acid, an analog thereof, an isomer thereof, and a derivative thereof. Preferred examples thereof include D- or L-ascorbic acid and a sugar derivative thereof (for example, $\gamma$-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly preferred examples thereof include D-, L-, or D,L-ascorbic acid (and an alkali metal salt thereof) or isoascorbic acid (or an alkali metal salt thereof), and a sodium salt is a preferred salt. A mixture of these reducing agents can be used as necessary.

Due to the reason that the effects and the like of the present invention are more excellent, the reducing agent solution is preferably allowed to flow so that the angle between the spreading direction in the spreading step and the spreading direction of the reducing agent solution is 0 degrees to 150 degrees, and more preferably allowed to flow so that the angle between the spreading direction in the spreading step and the spreading direction of the reducing agent solution is 0 degrees to 135 degrees.

Examples of the method of regulating the angle between the spreading direction in the spreading step and the spreading direction of the reducing agent solution include the method described in Examples of JP2009-150869A.

<Silver amplification solution>

The silver amplification solution is a solution containing a compound containing silver ions. As the compound containing silver ions, it is possible to use, for example, organic silver salts, inorganic silver salts, or silver complexes. Preferred examples thereof include silver ion-containing compounds having a high solubility in a solvent such as water, such as silver nitrate, silver acetate, silver lactate, silver butyrate, and silver thiosulfate. Silver nitrate is particularly preferable. The silver complex is preferably a silver complex in which silver is coordinated with ligands having a water-soluble group such as a hydroxyl group or a sulfone group, and examples thereof include silver hydroxythioether.

As the silver, the organic silver salt, the inorganic silver salt, or the silver complex is preferably contained in the silver amplification solution at a concentration of 0.001 mol/L to 5 mol/L, preferably 0.005 mol/L to 3 mol/L, and more preferably 0.01 mol/L to 1 mol/L.

Examples of the auxiliary agent of the silver amplification solution include a buffer, a preservative such as an antioxidant or an organic stabilizer, and a rate regulating agent. As the buffer, it is possible to use, for example, a buffer formed of acetic acid, citric acid, sodium hydroxide, or one of salts of these compounds, or formed of tris(hydroxymethyl) aminomethane, or other buffers that are used in general chemical experiments. These buffers are appropriately used to adjust the pH of the amplification solution to an optimum pH thereof. In addition, as the antifogging agent, an alkyl amine can be used as an auxiliary agent, and dodecyl amine is particularly preferable. In addition, a surfactant can be used for the intended purpose of improving the solubility of this auxiliary agent, and $C_9H_{19}$-$C_6H_4$-O-$(CH_2CH_2O)_{50}$H is particularly preferable.

Due to the reason that the effects and the like of the present invention are more excellent, the silver amplification solution is preferably allowed to flow from the direction opposite to the spreading direction in the spreading step described above and more preferably allowed to flow so that the angle between the spreading direction in the spreading step and the spreading direction of the silver amplification solution is 45 degrees to 180 degrees.

Examples of the method of regulating the angle between the spreading direction in the spreading step and the spreading direction of the silver amplification solution include the method described in Examples of JP2009-150869A.

[2] Concentration jig (not claimed as such but useful for practising embodiments of the invention)

According to an embodiment of the present invention in the concentration step described above of the immunological test method a concentration jig that is used, and it is a concentration jig including a container that accommodates the above-described specific superabsorbent polymer, where the container has an incorporation part for incorporating the above-described antigen-containable solution and a discharge unit for discharging the above-described antigen-concentrated solution.

By using the concentration in the above-described concentration step, the recovery time of the concentrated solution is shortened and the concentration step can be completed in a short time. In addition to this, the antigen-concentrated solution can be obtained quantitatively, and the variation in the concentration rate for each measurement can be suppressed to a low level. Furthermore, since the concentration time, the concentration amount, and the concentration rate can be controlled, it is possible to carry out high reproducible concentration more easily and repeatedly.

Hereinafter, suitable aspects of the concentration jig used in the concentration step described above will be described.

[Suitable aspect 1]

A suitable aspect 1 of the concentration jig a is a concentration jig including a cylinder that is the above-described container and a piston that is insertable into the cylinder.

The above suitable aspect 1 will be described with reference to the drawing.

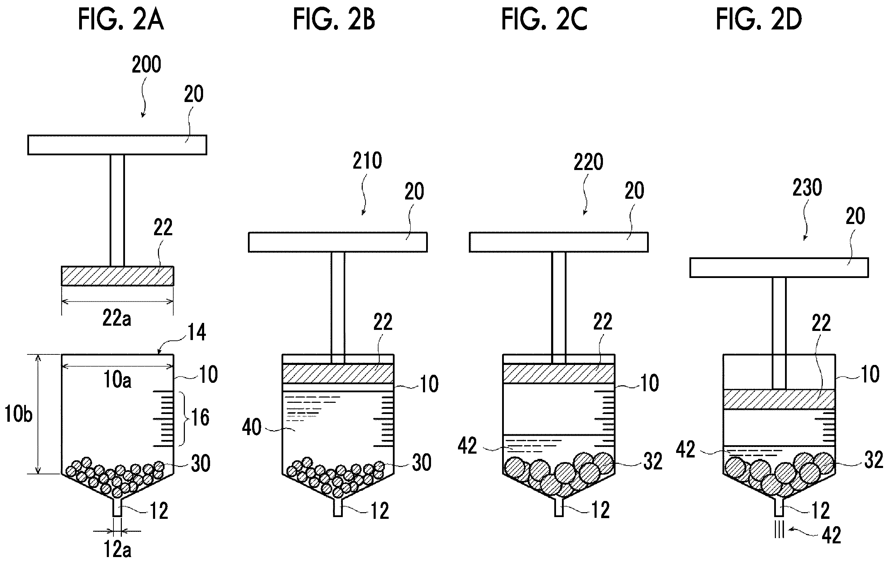

Fig. 2 is a schematic cross-sectional view illustrating a concentration jig that is one aspect of the above suitable aspect 1.

As illustrated in (A) of Fig. 2, a concentration jig 200 includes a cylinder 10 and a piston 20 that is insertable into the cylinder 10.

Here, the cylinder 10 has a nozzle part 12, an opening portion 14, and a gradation 16, and it accommodates a specific superabsorbent polymer 30.

[Procedure of concentration step]

First, an antigen-containable solution (a sample solution) 40 is incorporated into the cylinder 10.

Examples of the method of incorporating the sample solution 40 include (i) a method of plugging the nozzle part 12 and then placing the sample solution 40 in the cylinder 10 through the opening portion 14, and (ii) a method of inserting the piston 20 into the cylinder 10, subsequently immersing the nozzle part 12 in the sample solution 40, and incorporating the sample solution 40 into the cylinder 10 from the nozzle part 12 by pulling the piston in a state where the nozzle part 12 is immersed in the sample solution 40.

In the case of the above (i), the opening portion 14 serves as the above-described incorporation part. Alternatively, in the case of the above (ii), the nozzle part 12 serves as the above-described incorporation part.

In the above cases, the sample solution 40 is incorporated to reach a predetermined position of the cylinder 10 (for example, the upper end of the gradation 16). In this manner, a predetermined amount of the sample solution 40 can be incorporated into the cylinder 10 ((B) of Fig. 2).

In Fig. 2, the gradation 16 is marked on the cylinder 10; however, a stopper for incorporating a predetermined amount of the sample solution 40 may be provided instead of the gradation 16.

Next, the state of (B) of Fig. 2 is continued to be left for a predetermined time. As a result, mainly the liquid (particularly water) and impurities in the sample solution 40 are absorbed by the specific superabsorbent polymer 30, and the sample solution 40 is concentrated to become an antigen-concentrated solution 42 (the specific superabsorbent polymer 30 becomes a swollen specific superabsorbent polymer 32) ((C) of Fig. 2).

Then, the piston 20 in the state of (C) of Fig. 2 is pushed to discharge the antigen-concentrated solution 42 from the nozzle part 12 ((D) of Fig. 2). The discharged antigen-concentrated solution 42 is recovered to obtain the antigen-concentrated solution 42.

At the time of discharging the antigen-concentrated solution 42, a predetermined amount of the antigen-concentrated solution 42 is discharged by using the gradation 16 of the cylinder 10. Alternatively, the entire amount of the antigen-concentrated solution 42 is discharged. In this manner, a predetermined amount of the antigen-concentrated solution 42 can be recovered.

In Fig. 2, the gradation 16 is marked on the cylinder 10; however, a stopper for discharging a predetermined amount of the antigen-concentrated solution 42 may be provided instead of the gradation 16.

[Cylinder]

As described above, the cylinder 10 has the nozzle part 12, the opening portion 14, and the gradation 16, and it accommodates the specific superabsorbent polymer 30.

<Material>

The material of the cylinder 10 is not particularly limited; however, it is preferably a thermoplastic resin since thermoplastic resin can be subjected to injection molding, is inexpensive, and can be produced on a large scale. Specifically, it is preferably polypropylene, acryl, polyacetal, polyamide, polyethylene, polyethylene terephthalate, polycarbonate, polystyrene, polyphenylene sulfide, polybutylene terephthalate, polyvinyl chloride, an acrylonitrile-butadiene-styrene copolymer resin (an ABS resin), or an acrylonitrile-styrene copolymer resin (an AS resin) since this has a certain degree of hardness.

<Discharge unit>

The nozzle part 12 is a portion that discharges the antigen-concentrated solution 42.

The nozzle part 12 preferably has a structure in which the specific superabsorbent polymer 30 is not discharged. Examples of the method of forming such a structure include a method of making an inner diameter 12a of the nozzle part 12 smaller than a particle diameter 30a of the specific superabsorbent polymer 30 (before swelling) (preferably 1/1.5 or less of the particle diameter 30a, more preferably 1/2 or less of the particle diameter 30a, and still more preferably 1/5 or less of the particle diameter 30a), a method of installing a mesh for preventing the specific superabsorbent polymer 30 from being discharged from the nozzle part 12 (the pore diameter of the mesh is smaller than the particle diameter 30a of the specific superabsorbent polymer 30 (before swelling), preferably 1/1.5 or less of the particle diameter 30a, more preferably 1/2 or less of the particle diameter 30a, and still more preferably 1/5 or less of the particle diameter 30a), a method of plugging the nozzle part 12 (a plug is removed at the time of recovering the antigen-concentrated solution 42), and a method obtained by combining these.

The tip of the nozzle part 12 is preferably slanted from the viewpoint of easy recovery of the antigen-concentrated solution 42.

[Piston]

As described above, the concentration jig 200 includes the piston 20.

<Material>

The material of the piston 20 is not particularly limited, and the suitable aspect thereof is the same as that of the cylinder 10 described above. It is preferable that a rubber material (for example, silicone rubber) is attached to a tip part 22 of the piston 20, on a side to be inserted into the cylinder.

<Diameter>

A diameter 22a of the tip part 22 of the piston 20 is not particularly limited; however, it is preferably 5 times or more, more preferably 10 times or more, and still more preferably 20 times or more with respect to the particle diameter 30a of the specific superabsorbent polymer 30.

[Suitable aspect 2]

A suitable aspect 2 of the concentration jig according used in the concentration step as described above is a concentration jig including an inner tube which is the above-described container and an outer tube into which the inner tube is insertable and from which the inner tube is removable.
The suitable aspect 2 will be described with reference to the drawing.

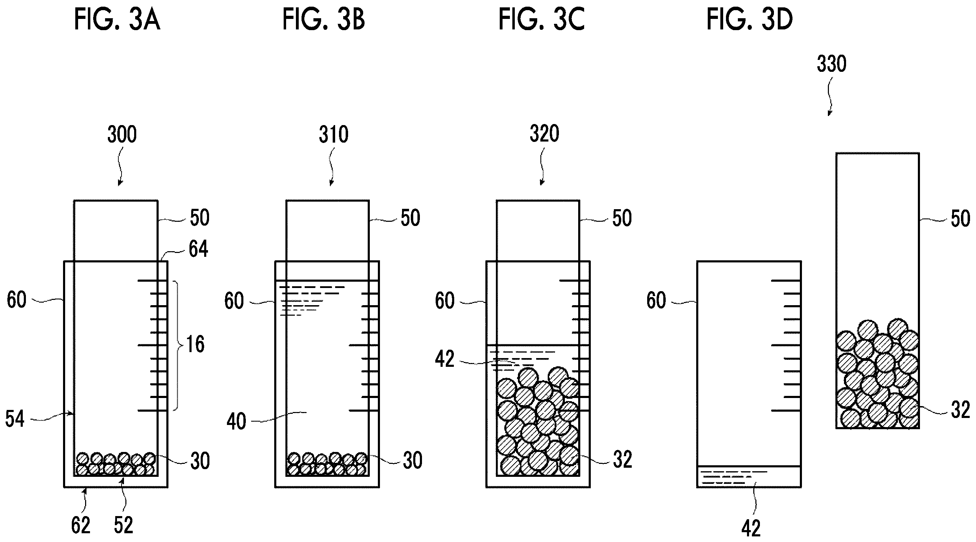

Fig. 3 is a schematic cross-sectional view illustrating an aspect of the suitable aspect 2.
As illustrated in (A) of Fig. 3, a concentration jig 300 includes an inner tube 50 and an outer tube 60 into which the inner tube 50 is insertable and from which the inner tube is removable.
Here, at least a part of a bottom surface 52 of the inner tube 50 has a mesh shape (not illustrated in the drawing). In addition, the inner tube 50 accommodates the specific superabsorbent polymer 30.
In addition, the outer tube 60 has the gradation 16. One end of the outer tube 60 is closed by a bottom surface 62, and the other end is opened.

[Procedure of concentration step]

First, the sample solution 40 is incorporated into the inner tube 50.
Examples of the method of incorporating the sample solution 40 include (i) a method of placing the sample solution 40 in the inner tube 50 through an opening portion of the inner tube 50 in a case where the inner tube 50 has the opening portion (not illustrated in the drawing) above the inner tube 50, and (ii) a method of placing the sample solution 40 through an opening portion 64 of the outer tube 60 in a state where the inner tube 50 is taken out of the outer tube 60 and then inserting the inner tube 50 into the outer tube 60 to incorporate the sample solution 40 into the inner tube 50 from a mesh-shaped portion of the inner tube 50.
In the case of the above (i), the opening portion of the inner tube 50 serves as the above-described incorporation part. Alternatively, in the case of the above (ii), the mesh-shaped portion of the inner tube 50 serves as the incorporation part.
In the above cases, the sample solution 40 is incorporated to reach a predetermined position of the outer tube 60 (for example, the upper end of the gradation 16). In this manner, a predetermined amount of the sample solution 40 can be incorporated into the inner tube 50 ((B) of Fig. 3).
Next, the state of (B) of Fig. 3 is continued to be left for a predetermined time. As a result, mainly the liquid (particularly water) and impurities in the sample solution 40 are absorbed by the specific superabsorbent polymer 30, and the sample solution 40 is concentrated to become an antigen-concentrated solution 42 (the specific superabsorbent polymer 30 becomes a swollen specific superabsorbent polymer 32) ((C) of Fig. 3).
Then, the inner tube 50 in the state of (C) of Fig. 3 is taken out to discharge the antigen-concentrated solution 42 from the mesh-shaped portion of the inner tube 50. The discharged antigen-concentrated solution 42 is collected in the outer tube 60. In this manner, a predetermined amount of the antigen-concentrated solution 42 can be recovered.

[Inner tube]

As described above, at least a part of the bottom surface 52 of the inner tube 50 has a mesh shape (not illustrated in the drawing). In addition, the inner tube 50 accommodates the specific superabsorbent polymer 30.
It is preferable that the entire bottom surface 52 of the inner tube 50 has a mesh shape. Further, it is preferable that a side surface 54 of the inner tube 50 also has a mesh shape; however, in a case where the sample solution 40 is incorporated by the method of the above (i), it is preferable that a portion (an upper portion) of the inner tube 50, which is exposed from the outer tube 60 when the inner tube 50 is inserted into the outer tube 60, does not have a mesh shape due to the reason that the sample solution 40 does not flow out from the inner tube 50 when the sample solution 40 is placed in the inner tube 50 through the opening portion of the inner tube 50.

<Material>

The material of the inner tube 50 is not particularly limited. However, due to the reason that the effects and the like of the present invention are more excellent, it is preferably a metal, a thermoplastic resin (a suitable aspect thereof is the same as that of the cylinder 10 described above), or a cloth, and more preferably a thermoplastic resin.

<Mesh>

Due to the reason that the effects and the like of the present invention are more excellent, the pore diameter of the mesh-shaped portion of the inner tube 50 is preferably smaller than the particle diameter 30a of the specific superabsorbent polymer 30 (before swelling), more preferably 1/1.5 or less of the particle diameter 30a, still more preferably 1/2 or less of the particle diameter 30a, and particularly preferably 1/5 or less of the particle diameter 30a.

Due to the reason that the effects and the like of the present invention are more excellent, the pore diameter of the mesh-shaped portion is preferably 0.1 $\mu$m to 5 mm and more preferably 0.2 $\mu$m to 2 mm.

[Outer tube]

As described above, the concentration jig 300 includes the outer tube 60.

<Material>

The material of the outer tube 60 is not particularly limited, and the suitable aspect thereof is the same as that of the cylinder 10 described above.

The inner tube 50 and/or the outer tube 60 may have a stopper so that the bottom surface 52 of the inner tube 50 and the bottom surface 62 of the outer tube 60 do not come into contact with each other.

[Suitable aspect 3]

A suitable aspect 3 of the concentration jig used in the concentration step as described above is a dropper-shaped concentration jig having a tube part and a pump part. Here, the tube part is the container described above.

The suitable aspect 3 will be described with reference to the drawing.

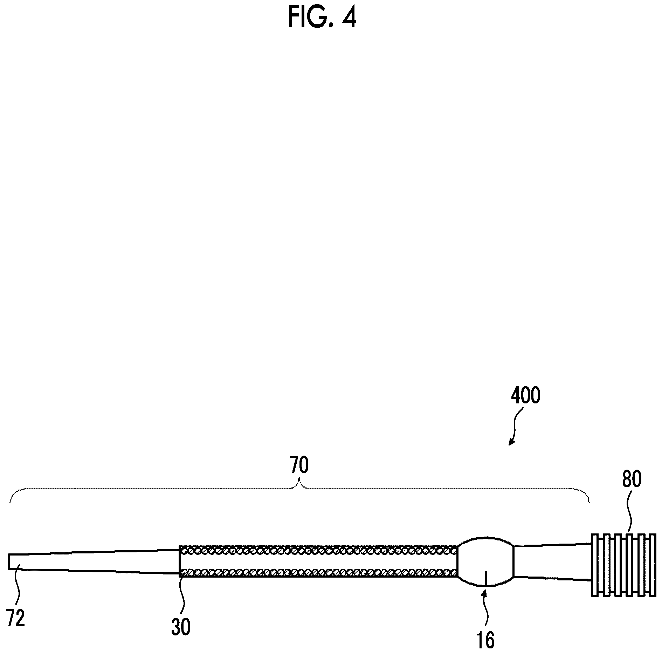

Fig. 4 is a schematic cross-sectional view illustrating an aspect of the suitable aspect 3.

As illustrated in Fig. 4, a concentration jig 400 is dropper-shaped and has a tube part 70 and a pump part 80.

Here, the tube part 70 has a nozzle part 72 and the gradation 16, and accommodates the specific superabsorbent polymer 30.

[Procedure of concentration step]

First, the sample solution 40 is incorporated into the tube part 70. Specifically, the nozzle part 72 is immersed in the sample solution 40 in a state where the pump part 80 is pressed, and the sample solution 40 is incorporated into the tube part 70 from the nozzle part 72 by releasing the pressing of the pump part 80 in a state where the nozzle part 72 is immersed in the sample solution 40.

At that time, the sample solution 40 is incorporated to reach a predetermined position (for example, the gradation 16) of the tube part 70. In this manner, a predetermined amount of the sample solution 40 can be incorporated into the tube part 70.

Next, a predetermined time is allowed to pass. As a result, mainly the liquid (particularly water) and impurities in the sample solution 40 are absorbed by the specific superabsorbent polymer 30, and the sample solution 40 is concentrated to become an antigen-concentrated solution (the specific superabsorbent polymer 30 becomes a swollen specific superabsorbent polymer).

Then, the pump part 80 is pressed to discharge the antigen-concentrated solution from the nozzle part 72. The discharged antigen-concentrated solution is recovered to obtain the antigen-concentrated solution.

At the time of discharging the antigen-concentrated solution, the antigen-concentrated solution is discharged so that the entire amount is discharged or the antigen-concentrated solution is discharged to reach a predetermined gradation (a gradation other than the gradation 16) (not illustrated in the drawing). In this manner, a predetermined amount of the antigen-concentrated solution can be recovered.

As described above, since the sample solution 40 is incorporated into the tube part 70 from the nozzle part 72 and the antigen-concentrated solution is discharged from the nozzle part 72, the nozzle part 72 serves as the above-described incorporation part and the above-described discharge unit.

[Tube part]

As described above, the tube part 70 has the nozzle part 72 and the gradation 16, and accommodates the specific superabsorbent polymer 30.

<Material>

The material of the tube part 70 is not particularly limited, and the suitable aspect thereof is the same as that of the cylinder 10 described above.

<specific superabsorbent polymer>

As described above, the tube part 70 accommodates the specific superabsorbent polymer 30.
The specific superabsorbent polymer 30 is preferable to be arranged to stay inside the tube part 70 and more preferable to be present on the inner wall of the tube part 70. Examples of the method of arranging the superabsorbent polymer 30 to stay inside the tube part 70 include a method of fixing the superabsorbent polymer 30 to the inner wall of the tube part 70 with an adhesive, a method of making the inner diameter of the nozzle 72 smaller than the particle diameter of the specific superabsorbent polymer 30, and a method of installing a mesh having a pore diameter smaller than the particle diameter of the specific superabsorbent polymer 30 in the nozzle part.
Further, it is preferable that the position where the specific superabsorbent polymer 30 is accommodated is closer to the nozzle part 72 than to a predetermined position (for example, the gradation 16) for incorporating the sample solution. Such an aspect makes it possible to bring the entire accommodated specific superabsorbent polymers 30 into contact with the sample solution 40, and thus it is possible to achieve a concentration rate having good reproducibility.

[Pump part]

As described above, the concentration jig 400 includes the pump part 80.

<Material>

The material of the pump part 80 is not particularly limited, and the suitable aspect thereof is the same as that of the cylinder 10 described above.
In addition to the tube part 70, the pump part 80 may accommodate the specific superabsorbent polymer 30; however, in such a case, it is preferable that the sample solution 40 is incorporated so that the sample solution 40 comes into contact with the specific superabsorbent polymer 30 accommodated in the pump part 80.

Examples

Hereinafter, the present invention will be described in more detail with reference to Examples; however, the present invention is not limited thereto.

[A] Example in which antigen is influenza virus

[Preparation of sample solution]

A sample solution (an antigen-containable solution) was prepared using a Quick S-Influ A·B "SEIKEN" negative/positive control solution (product number: 322968, manufactured by DENKA SEIKEN Co., Ltd.).
Specifically, the above positive control solution was subjected to serial 10-time dilutions with a phosphate buffered salts (PBS) buffer containing 0.1% by mass bovine serum albumin (BSA), and sample solutions (antigen-containable solutions) with the respective dilution rates were prepared.

[Example A1]

Immunochromatography of Example A1 was carried out as follows.

[Concentration step]

Commercially available superabsorbent polymer (SAP) particles (model number: 197-12451, manufactured by FUJIFILM Wako Pure Chemical Corporation) were graded to obtain a superabsorbent polymer that would be used in the concentration step. The superabsorbent polymer had a particle diameter of 0.05 mm, a swelling ratio of 600 g/g, and a water absorption rate of 20 g/min.

1 g of the superabsorbent polymer was added dropwise to 6 mL of the sample solution described above. After the dropwise addition, the resultant mixture was stirred with a spatula for about 10 seconds and allowed to stand. After a standing time of 10 minutes, a solution (an influenza virus-concentrated solution) (an antigen-concentrated solution) that was not absorbed by the superabsorbent polymer was recovered with a pipette (manufactured by Eppendorf).

[Spreading step]

The nitrocellulose membrane 100 as illustrated in Fig. 1 was prepared, which has from the upstream side; the gold colloid holding pad 1, the test line 2, the control line 3, and the coloring reagent immobilization line 4. The gold colloid holding pad 1 is a pad that holds gold colloids (modified gold particles) modified with an anti-influenza A type monoclonal antibody, the test line 2 is a line on which the anti-influenza A type monoclonal antibody is immobilized, the control line 3 is a line for checking the spreading, and the coloring reagent immobilization line 4 is a line for detecting the reducing agent solution described later.

The above influenza virus-concentrated solution was dropwise added onto the gold colloid holding pad. As a result, gold particle composite bodies, which are composite bodies of the influenza A type virus in the solution and the gold colloid particles (modified gold particles) modified with the anti-influenza A type monoclonal antibody in the gold colloid holding pad, were formed. In this state, the gold particle composite bodies were spread toward the downstream side of the nitrocellulose membrane.

[Capturing step]

The gold particle composite bodies that are spread in the spreading step is captured on the test line.

[Silver amplification step]

The silver amplification step was carried out as follows.

<Preparation of reducing agent solution>

23.6 mL of an aqueous solution of 1 mol/L iron nitrate, which was produced by dissolving iron (III) nitrate nonahydrate (manufactured by FUJIFILM Wako Pure Chemical Corporation) in water, and 13.1 g of citric acid (manufactured by FUJIFILM Wako Pure Chemical Corporation) were dissolved in 290 g of water. After all of the substances were dissolved, 36 mL of nitric acid (10% by mass) was added thereto while stirring with a stirrer, 60.8 g of ammonium iron (II) sulfate hexahydrate (manufactured by FUJIFILM Wako Pure Chemical Corporation) was added thereto, and the resultant solution was used as the reducing agent solution.

<Preparation of silver amplification solution>

8 mL of a silver nitrate solution (including 10 g of silver nitrate) and 24 mL of an aqueous solution of 1 mol/L iron nitrate were added to 66 g of water. Further, this solution was mixed with a solution obtained by dissolving 5.9 mL of nitric acid (10% by mass), 0.1 g of dodecyl amine (manufactured by FUJIFILM Wako Pure Chemical Corporation), and 0.1 g of a surfactant $C_{12}H_{25}$-$C_6H_4$-O-$(CH_2CH_2O)_{50}$H in 47.6 g of water in advance, and the resultant solution was used as the silver amplification solution.

<Spreading of reducing agent solution>

In the nitrocellulose membrane, the reducing agent solution prepared as described above was allowed to flow from the same direction as that of the spreading step described above (from the upstream side).

<Spreading of silver amplification solution>

After the coloring reagent immobilization line was discolored, the silver amplification solution prepared as described above was allowed to flow from the direction opposite to the spreading direction (from the downstream side) in the spreading step. In this manner, the gold particle composite body captured on the test line was silver amplified.

[Evaluation]

The coloration of the test line was visually checked, and the lowest dilution rate (the minimum detection sensitivity) at which coloration was confirmed was investigated. The results are shown in Table 1. It means that as the minimum detection sensitivity is lower, an antigen can be detected even in a specimen having a low antigen concentration, which means the detection sensitivity is high.

[Example A2]

Immunochromatography was carried out and evaluated according to the same procedure as in Example A1 except that in the concentration step, the following superabsorbent polymer was used. The results are shown in Table 1.

[superabsorbent polymer used in Example A2]

Commercially available superabsorbent polymer (SAP) particles (manufactured by M2 Polymer Technologies Inc.; SAP Sphere 2.5 mm) were graded to obtain a superabsorbent polymer that would be used in the concentration step. The superabsorbent polymer had a particle diameter of 2.5 mm, a swelling ratio of 13 g/g, and a water absorption rate of 0.5 g/min.

[Example A3]

Immunochromatography was carried out and evaluated according to the same procedure as in Example A1 except that in the concentration step, the following superabsorbent polymer was used. The results are shown in Table 1.

[superabsorbent polymer used in Example A3]

Superabsorbent polymer (SAP) particles were prepared with reference to the methods of Example 1 and Comparative Example 1 disclosed in JP2015-536375A, and for particles having a different particle diameter, classification was carried out using a 0.5 mm sieve to obtain superabsorbent polymer particles having a particle diameter of 0.5 mm and a swelling ratio of 30 g/g.

[Example A4]

Immunochromatography was carried out and evaluated according to the same procedure as in Example A1 except that in the concentration step, the following superabsorbent polymer was used. The results are shown in Table 1.

[superabsorbent polymer used in Example A4]

The superabsorbent polymer was prepared according to the same procedure except that a 0.1 mm sieve was used in the preparation of the superabsorbent polymer used in Example A3. As a result, superabsorbent polymer particles having a particle diameter of 0.1 mm and a swelling ratio of 90 g/g were obtained.

[Comparative Example A1]

Immunochromatography was carried out and evaluated according to the same procedure as in Example A1 except that without carrying out the concentration step, the above-described sample solution itself was used instead of the influenza virus-concentrated solution in the spreading step. The results are shown in Table 1.

[Comparative Example A2]

Immunochromatography was carried out and evaluated according to the same procedure as in Example A1 except that in the concentration step, the following superabsorbent polymer was used.
As a result, coloration could not be confirmed in Comparative Example A2. It is presumed to be because the antibody-antigen reaction was inhibited by the impurities.

[superabsorbent polymer used in Comparative Example A2]

Polyacrylic acid 5000 manufactured by FUJIFILM Wako Pure Chemical Corporation was graded to obtain a superabsorbent polymer that would be used in the concentration step. The superabsorbent polymer had a particle diameter of 0.01 mm, a swelling ratio of 1,000 g/g, and a water absorption rate of 60 g/min.

[Comparative Example A3]

Immunochromatography was carried out and evaluated according to the same procedure as in Example A1 except that in the concentration step, the following superabsorbent polymer was used. The results are shown in Table 1.

[superabsorbent polymer used in Comparative Example A3]

A polyvinyl alcohol (PVA) solution of 10% by mass was prepared and this solution was dropwise added to 1M HCl to prepare particles. The obtained particles were graded and used as the superabsorbent polymer that would be used in the concentration step. The superabsorbent polymer had a particle diameter of 0.5 mm, a swelling ratio of 0.2 g/g, and a water absorption rate of 0.01 g/min.

[Table 1]

| | Comparative Example A1 | Comparative Example A2 | Example A1 | Example A2 | Example A3 | Example A4 | Comparative Example A3 |
|---|---|---|---|---|---|---|---|
| Concentration step | Absent | Present | Present | Present | Present | Present | Present |
| Swelling ratio [g/g] | - | 1,000 | 600 | 13 | 30 | 90 | 0.2 |
| Particle diameter [mm] | - | 0.01 | 0.05 | 2.5 | 0.5 | 0.1 | 0.5 |
| Minimum detection sensitivity | 1/320 | - | 1/2560 | 1/1600 | 1/3200 | 1/3200 | 1/320 |

As can be seen from Table 1 and the like, as compared with Comparative Example A1 in which the concentration step was not carried out and Comparative Examples A2 and A3 in which the concentration step was carried out using a superabsorbent polymer of which the swelling ratio was out of a specific range, Examples A1 to A4 in which the concentration step was carried out using a superabsorbent polymer of which the swelling ratio was in the specific range (a specific superabsorbent polymer) exhibited high detection sensitivity. Among them, Examples A1 and Examples A3 and A4, in which the swelling ratio of the specific superabsorbent polymer was 20 g/g or more, exhibited higher detection sensitivity. Among them, Examples A3 and A4 in which the swelling ratio of the specific superabsorbent polymer was 500 g/g or less exhibited further higher detection sensitivity.

[B] Case where antigen is LAM

[Preparation of sample solution]

Lipoarabinomannan (LAM) (02249-61, Nacalai Tesque, Inc.) extracted from tubercle bacillus was added to a urine sample obtained by pooling urine samples (BioIVT LLC) of healthy subjects to prepare a sample solution (an antigen-containable solution) of a LAM concentration shown in Table 2.

[Example B1]

Immunochromatography of Example B1 was carried out as follows.

[Concentration step]

1 g of the same superabsorbent polymer as the superabsorbent polymer used in the above-described Example A1 was dropwise added to 6 mL of the above-described sample solution. After the dropwise addition, the resultant mixture was stirred for 10 seconds and allowed to stand. After a standing time of 10 minutes, a solution (a LAM-concentrated solution) (an antigen-concentrated solution) that was not absorbed by the superabsorbent polymer was recovered with a pipette (manufactured by Eppendorf). The concentration of urea in the LAM-concentrated solution was 5 times or less with respect to the concentration of urea in the sample solution.

[Spreading step]

The nitrocellulose membrane 100 as illustrated in Fig. 1 was prepared, which has from the upstream side; the gold colloid holding pad 1, the test line 2, the control line 3, and the coloring reagent immobilization line 4. The gold colloid holding pad 1 is a pad that holds gold colloids (modified gold particles) modified with an anti-LAM monoclonal antibody, the test line 2 is a line on which the anti-LAM monoclonal antibody is immobilized, the control line 3 is a line for checking the spreading, and the coloring reagent immobilization line 4 is a line for detecting the reducing agent solution in the silver amplification step described later.

The above LAM-concentrated solution was dropwise added onto the gold colloid holding pad. As a result, gold particle composite bodies, which are composite bodies of the LAM in the solution and the gold colloid particles (modified gold particles) modified with the anti-LAM monoclonal antibody, were formed. In this state, the gold particle composite bodies were spread from the upstream side toward the downstream side of the nitrocellulose membrane.

[Capturing step]

The gold particle composite bodies that are spread in the spreading step is captured on the test line.

[Silver amplification step]

The silver amplification step was carried out according to the same procedure as in Example A1.
In this manner, the gold particle composite body captured on the test line was silver amplified.

[Evaluation]

The coloration of the test line was visually checked and evaluated according to the following criteria.

+++: The coloration is strong.
++: The coloration is observed.
+: The coloration is weak.
-: No coloration is observed.

The results are shown in Table 2. It means that the lower the lowest LAM concentration (the minimum detection sensitivity) among the LAM concentrations of the sample solution evaluated as +++, ++, or +, the higher the detection sensitivity.

[Example B2]

Immunochromatography was carried out and evaluated according to the same procedure as in Example B1 except that in the concentration step, the same superabsorbent polymer as the superabsorbent polymer used in Example A2 described above was used. The results are shown in Table 2. The concentration of urea in the LAM-concentrated solution was 5 times or less with respect to the concentration of urea in the sample solution.

[Example B3]

Immunochromatography was carried out and evaluated according to the same procedure as in Example B1 except that in the concentration step, the same superabsorbent polymer as the superabsorbent polymer used in Example A3 described above was used. The results are shown in Table 2. The concentration of urea in the LAM-concentrated solution was 5 times or less with respect to the concentration of urea in the sample solution.

[Example B4]

Immunochromatography was carried out and evaluated according to the same procedure as in Example B1 except that in the concentration step, the same superabsorbent polymer as the superabsorbent polymer used in Example A4 described above was used. The results are shown in Table 2. The concentration of urea in the LAM-concentrated solution was 5 times or less with respect to the concentration of urea in the sample solution.

[Comparative Example B1]

Immunochromatography was carried out and evaluated according to the same procedure as in Example B1 except that without carrying out the concentration step, the above-described sample solution itself was used instead of the LAM-concentrated solution in the spreading step. The results are shown in Table 2.

[Comparative Example B2]

Immunochromatography was carried out and evaluated according to the same procedure as in Example B1 except that in the concentration step, the same superabsorbent polymer as the superabsorbent polymer used in Comparative Example A2 described above was used. The results are shown in Table 2.
The coloration could not be confirmed in Comparative Example B2. It is presumed to be because the antibody-antigen reaction was inhibited by the impurities such as urea.

[Comparative Example B3]

Immunochromatography was carried out and evaluated according to the same procedure as in Example B1 except that in the concentration step, the same superabsorbent polymer as the superabsorbent polymer used in Comparative Example A3 described above was used. The results are shown in Table 2.

[Table 2]

| LAM concentration [ng/mL] | Comparative Example B1 | Comparative example B2 | Example B1 | Example B2 | Example B3 | Example B4 | Comparative example B3 |
|---|---|---|---|---|---|---|---|
| 0.2 | ++ | - | ++ | ++ | ++ | ++ | ++ |
| 0.1 | + | - | ++ | ++ | ++ | ++ | + |
| 0.05 | + | - | ++ | ++ | ++ | ++ | + |
| 0.025 | - | - | ++ | ++ | ++ | ++ | - |
| 0.0125 | - | - | + | + | ++ | ++ | - |
| 0.010 | - | - | + | + | ++ | ++ | - |
| 0.005 | - | - | + | - | + | + | - |
| 0.0025 | - | - | - | - | + | + | - |
| 0.0005 | - | - | - | - | - | - | - |
| 0 | - | - | - | - | - | - | - |

As can be seen from Table 2 and the like, as compared with Comparative Example B1 in which the concentration step was not carried out and Comparative Examples B2 and B3 in which the concentration step was carried out using a superabsorbent polymer of which the swelling ratio was out of a specific range, Examples B1 to B4 in which the concentration step was carried out using a superabsorbent polymer of which the swelling ratio was in the specific range (a specific superabsorbent polymer) exhibited high detection sensitivity. Among them, Examples B1 and Examples B3 and B4, in which the swelling ratio of the specific superabsorbent polymer was 20 g/g or more, exhibited higher detection sensitivity. Among them, Examples B3 and B4 in which the swelling ratio of the specific superabsorbent polymer was 500 g/g or less exhibited further higher detection sensitivity.

[C] False positiveness evaluation

[False positiveness evaluation 1]

Individual urines (BioIVT LLC) to which LAM was not added were concentrated by the method of Example B1 and evaluated in the same manner. Five individual urines were used for evaluation by the above method and examined for the concern of false positiveness. The results are shown in Table 3.

+: The coloration is observed.

-: No coloration is observed.

[False positiveness evaluation 2]

Individual urine (BioIVT LLC) to which LAM was not added was concentrated by the method of Example B1 and evaluated in the same manner as in Example B1 except that an anti-LAM polyclonal antibody was used instead of the anti-LAM monoclonal antibody. Five individual urines were used for evaluation by the above method and examined for the concern of false positiveness. The results are shown in Table 3.

+: The coloration is observed.
-: No coloration is observed.

[Table 3]

| Individual urine No. | False positiveness evaluation 1 | False positiveness evaluation 2 |
|---|---|---|
| 1 | - | - |
| 2 | - | - |
| 3 | - | - |
| 4 | - | + |
| 5 | - | - |

As can be seen from Table 3, no false positiveness was observed in a case where the monoclonal antibody was used; however, false positiveness was observed in a case where the polyclonal antibody was used.

[D] Immunochromatography using concentration jig

As described below, immunochromatography was carried out using a concentration jig in the concentration step.

[Preparation of concentration jig]

The syringe-shaped concentration jig 200 as illustrated in (A) of Fig. 2 was prepared.
Here, regarding the cylinder 10, the inner diameter 10a is 29 mmφ, the length 10a is 100 mm, and the inner diameter 12a of the nozzle part 12 is 1 mmφ. In addition, the cylinder 10 accommodates the same superabsorbent polymer (1 g) as the superabsorbent polymer used in the above-described Example A1. In addition, the nozzle part 12 is plugged.
Further, silicone rubber is attached to a tip part 22 of a piston 20.

[Example D1]

Immunochromatography was carried out and evaluated according to the same procedure as in Example A1 except that the concentration step was carried out as follows. As a result, a high detection sensitivity was exhibited as in Example A1.

[Concentration step]

6 mL of the above-described sample solution (the sample solution 40) was added with a pipette from the opening portion 14 of the concentration jig 200 prepared as described above ((B) of Fig. 2).
Next, the state of (B) of Fig. 2 was continued to be left for 10 minutes. As a result, mainly water and impurities in the sample solution were absorbed by the superabsorbent polymer, and the sample solution became the concentrated antigen-concentrated solution 42. Then, the piston 20 was installed in the cylinder 10 through the opening portion 14 ((C) of Fig. 2).

Then, the nozzle part 12 in the state of (C) of Fig. 2 was unplugged, and the piston 20 was pushed to recover 200 μL of the antigen-concentrated solution 42 from the nozzle part 12 ((D) of Fig. 2).

[Recovery time]

In the concentration step of Example D1, the time (the recovery time) required to recover the antigen-concentrated solution 42 was measured. It is noted that the above concentration step was carried out 10 times in total, and the recovery time was measured each time. Then, the average of the recovery times (the average recovery time) was determined.

In addition, the average recovery time was similarly determined for Example A1 described above.

The results are shown in Table 4 below.

[Coefficient of variation of concentration rate]

The concentration rate in the concentration step of Example D1 was evaluated. The concentration rate was evaluated using test solutions subjected to 2-time dilution, 4-time dilution, 8-time dilution, 10-time dilution, 16-time dilution, 20-time dilution, 32-time dilution, and so on. A dilution factor of a sample solution after concentration, at which the test solution is detectable, was examined as compared with a dilution factor of a sample solution before concentration, at which the test solution is detectable, whereby the concentration rate of each of the repeated experiments was calculated.

Concentration rate = (detectable dilution factor after concentration)/(detectable dilution factor before concentration)

The concentration rates of the liquids recovered by the method of Example A1 and the method of Example D1 were evaluated at n = 10.

The coefficient of variation of the result at n = 10 can be derived from the following expression.

Standard deviation σ:

$$\sigma = \sqrt{\{(1/N)\Sigma(x\_av. - xi)^2\}}$$

Coefficient of variation CV:

$$CV = \sigma/x\_av.$$

(here, x_av. is the average value of 10 measurements)

[Table 4]

|  | Example A1 | Example D1 |
|---|---|---|
| Average recovery time | 1 minute | 20 seconds |
| Coefficient of variation of concentration rate | 0.59 | 0.28 |

As can be seen from Table 4, the average recovery time was 1 minute in the method of Example A1; however, the average recovery time was shortened to 20 seconds by using the syringe-shaped concentration jig, and it was found that the antigen-concentrated solution can be recovered in a shorter time. In addition, regarding the variation in influenza virus concentration after concentration when the same operation had been repeated 10 times, the coefficient of variation of the concentration rate was reduced from 60% to 30%, and thus it was found that the variation for each measurement is suppressed to a low level and the method is excellent in measurement accuracy.

Further, when the concentration step was similarly carried out using the concentration jig illustrated in Fig. 3 and the concentration jig illustrated in Fig. 4, and the average recovery time and the coefficient of variation of the concentration rate were determined, it was confirmed that similar to Example D1, the antigen-concentrated solution can be recovered in a shorter time and the method is excellent in measurement accuracy.

In a manner similar to the above-described immunochromatography carried out as described above using the concentration jig, when the concentration jig was prepared, immunochromatography was carried out according to the same procedure as in Example B1, and the recovery time and the coefficient of variation of the concentration rate of the antigen-concentrated solution were determined in the concentration step and the immunochromatography carried out in Example D1, the same effect was confirmed in the detection of the lipoarabinomannan (LAM) concentration in the urine sample.

Explanation of References

1: gold colloid holding pad
2: test line
3: control line
4: coloring reagent immobilization line
100: nitrocellulose membrane
10: cylinder
12: nozzle part
14: opening portion
16: gradation
20: piston
22: tip part
30: specific superabsorbent polymer
32: swollen specific superabsorbent polymer
40: antigen-containable solution (sample solution)
42: antigen-concentrated solution
50: inner tube
52: bottom surface
54: side surface
60: outer tube
62: bottom surface
64: opening portion
70: tube part
72: nozzle part
80: pump part
200, 210, 220, 230, 300, 310, 320, 330, 400: concentration jig

**Claims**

1. An immunological test method comprising:

   a concentration step of concentrating an antigen-containable solution by mixing the antigen-containable solution with a superabsorbent polymer (30) to obtain an antigen-concentrated solution by incorporating impurities including urea contained in the antigen-containable solution together with water into the superabsorbent polymer (30); and
   a detection step of detecting an antigen in the antigen-concentrated solution using an antigen-antibody reaction, wherein the swelling ratio of the superabsorbent polymer (30) ranges from including 20 g/g to including 500 g/g, wherein the swelling ration is defined as grams of water retained by one gram of the superabsorbent polymer, and an antibody that is used in the antigen-antibody reaction is a monoclonal antibody,
   wherein the superabsorbent polymer is a polyacrylic acid-based, polyacrylamide-based, cellulose-based, or polyethylene oxide-based polymer,
   wherein the antigen-containable solution is urine,
   wherein a concentration of urea in the antigen-concentrated solution is 5 times or less with respect to a concentration of urea in the antigen-containable solution.

2. The immunological test method according to claim 1,
   wherein a particle diameter of the superabsorbent polymer is 5 mm or less.

3. The immunological test method according to claim 1 or 2,
   wherein a water absorption rate of the superabsorbent polymer is 0.01 g/min or more and 40 g/min or less per 1 g of the superabsorbent polymer.

4. The immunological test method according to any one of claims 1 to 3,
wherein the immunological test method is immunochromatography.

5. The immunological test method according to claim 4,

wherein the detection step include;
a spreading step of spreading gold particle composite bodies on an insoluble carrier having a reaction site at which a second binding substance capable of binding to an antigen in the antigen-concentrated solution has been immobilized, in a state where the gold particle composite bodies which are composite bodies of the antigen and modified gold particles which are gold particles modified with a first binding substance capable of binding to the antigen are formed,
a capturing step of capturing the gold particle composite bodies at the reaction site of the insoluble carrier, and
a silver amplification step of silver-amplifying the gold particle composite body captured in the capturing step.

6. The immunological test method according to any one of claims 1 to 5, wherein in the concentration step a concentration jig (200, 300, 400) is used that comprises:

a container containing the superabsorbent polymer,
wherein the container has an incorporation part configured for incorporating the antigen-containable solution and a discharge unit configured for discharging the antigen-concentrated solution.

7. The immunological test method according to claim 6, wherein the concentration jig (200) comprises:

a cylinder (10) that is the container; and
a piston (20) that is insertable into the cylinder.

8. The immunological test method according to claim 6, wherein the concentration jig (300) comprises:

an inner tube (50) that is the container; and
an outer tube (60) into which the inner tube (50) is insertable and from which the inner tube is removable.

9. The immunological test method according to claim 6,

wherein the concentration jig (400) has a dropper shape having a tube part (70) and a pump part (80), and the tube part (70) is the container.

**Patentansprüche**

1. Immunologischer Testverfahren, umfassend:

einen Konzentrationsschritt des Konzentrierens einer Lösung, die Antigen enthalten kann, durch Mischen der Lösung, die Antigen enthalten kann, mit einem superabsorbierenden Polymer (30), um eine antigenkonzentrierte Lösung durch Aufnehmen von Verunreinigungen, die Harnstoff enthalten, der in der Lösung, die Antigen enthalten kann, enthalten ist, zusammen mit Wasser in das superabsorbierende Polymer (30) zu erhalten; und
einen Detektionsschritt des Detektierens eines Antigens in der antigenkonzentrierten Lösung unter Verwendung einer Antigen-Antikörper-Reaktion,
wobei das Quellverhältnis des superabsorbierenden Polymers (30) im Bereich von einschließlich 20 g/g bis einschließlich 500 g/g liegt, wobei das Quellverhältnis als Gramm Wasser, das durch ein Gramm des superabsorbierenden Polymers zurückgehalten wird, definiert ist, und
ein Antikörper, der bei der Antigen-Antikörper-Reaktion verwendet wird, ein monoklonaler Antikörper ist,
wobei das superabsorbierende Polymer ein Polymer auf Polyacrylsäurebasis, Polyacrylamidbasis, Cellulosebasis oder Polyethylenoxidbasis ist,
wobei die Lösung, die Antigen enthalten kann, Urin ist,
wobei eine Konzentration von Harnstoff in der antigenkonzentrierten Lösung in Bezug auf eine Konzentration von Harnstoff in der Lösung, die Antigen enthalten kann, das 5-fache oder weniger beträgt.

**2.** Immunologisches Testverfahren nach Anspruch 1,
wobei ein Partikeldurchmesser des superabsorbierenden Polymers 5 mm oder weniger beträgt.

**3.** Immunologisches Testverfahren nach Anspruch 1 oder 2,
wobei eine Wasserabsorptionsrate des superabsorbierenden Polymers 0,01 g/min oder mehr und 40 g/min oder weniger pro 1 g des superabsorbierenden Polymers beträgt.

**4.** Immunologisches Testverfahren nach einem der Ansprüche 1 bis 3,
wobei das immunologische Testverfahren Immunochromatographie ist.

**5.** Immunologisches Testverfahren nach Anspruch 4,

wobei der Detektionsschritt umfasst;
einen Verbreitungsschritt des Verbreitens von zusammengesetzten Körpern von Goldteilchen auf einen unlöslichen Träger, der eine Reaktionsstelle, an der eine zweite Bindungssubstanz, die in der Lage ist, an ein Antigen in der antigenkonzentrierten Lösung zu binden, immobilisiert wurde, aufweist, in einem Zustand, in dem die zusammengesetzten Körper von Goldteilchen gebildet sind, die zusammengesetzte Körper aus dem Antigen und modifizierten Goldteilchen, die Goldteilchen, die mit einer ersten Bindungssubstanz, die in der Lage ist, sich an das Antigen zu binden, modifiziert wurden, sind, sind,
einen Einfangschritt des Einfangens der zusammengesetzten Körper von Goldteilchen an der Reaktionsstelle des unlöslichen Trägers, und
einen Silberverstärkungsschritt des Verstärkens von Silber des bei dem Einfangschritt eingefangenen zusammengesetzten Körpers von Goldteilchen.

**6.** Immunologisches Testverfahren nach einem der Ansprüche 1 bis 5, wobei bei dem Konzentrationsschritt eine Konzentrationsvorrichtung (200, 300, 400) verwendet wird, die umfasst:

einen Behälter, der das superabsorbierende Polymer enthält,
wobei der Behälter einen Aufnahmeteil, der zum Aufnehmen der Lösung, die Antigen enthalten kann, konfiguriert ist, und eine Abgabeeinheit, die zum Abgeben der antigenkonzentrierten Lösung konfiguriert ist, aufweist.

**7.** Immunologisches Testverfahren nach Anspruch 6, wobei die Konzentrationsvorrichtung (200) umfasst:

einen Zylinder (10), der der Behälter ist; und
einen Kolben (20), der in den Zylinder einführbar ist.

**8.** Immunologisches Testverfahren nach Anspruch 6, wobei die Konzentrationsvorrichtung (300) umfasst:

ein Innenrohr (50), das der Behälter ist; und
ein Außenrohr (60), in das das Innenrohr (50) einführbar ist und aus dem das Innenrohr entfernbar ist.

**9.** Immunologisches Testverfahren nach Anspruch 6,

wobei die Konzentrationsvorrichtung (400) eine Tropfform mit einem Rohrteil (70) und einem Pumpenteil (80) aufweist, und
der Rohrteil (70) der Behälter ist.

**Revendications**

**1.** Procédé d'essai immunologique comprenant :

une étape de concentration consistant à concentrer une solution contenant un antigène en mélangeant la solution contenant un antigène avec un polymère superabsorbant (30) pour obtenir une solution concentrée en antigène en incorporant des impuretés incluant de l'urée contenue dans la solution contenant un antigène ainsi que de l'eau dans le polymère superabsorbant (30) ; et
une étape de détection consistant à détecter un antigène dans la solution concentrée en antigène en utilisant une réaction antigène -anticorps,

dans lequel le taux de gonflement du polymère superabsorbant (30) varie de 20 g/g inclus à 500 g/g inclus, dans lequel le taux de gonflement est défini comme des grammes d'eau retenus par un gramme du polymère superabsorbant, et

un anticorps qui est utilisé dans la réaction antigène -anticorps est un anticorps monoclonal,

dans lequel le polymère superabsorbant est un polymère à base d'acide polyacrylique, à base de polyacrylamide, à base de cellulose ou à base d'oxyde de polyéthylène,

dans lequel la solution contenant un antigène est urine,

dans lequel une concentration d'urée dans la solution concentrée en antigène est 5 fois ou moins par rapport à une concentration d'urée dans la solution contenant un antigène.

2. Procédé d'essai immunologique selon la revendication 1,
dans lequel un diamètre de particule du polymère superabsorbant est de 5 mm ou moins.

3. Procédé d'essai immunologique selon la revendication 1 ou la revendication 2,
dans lequel un taux d'absorption d'eau du polymère superabsorbant est de 0,01 g/min ou plus et de 40 g/min ou moins par 1 g du polymère superabsorbant.

4. Procédé d'essai immunologique selon l'une quelconque des revendications 1 à 3,
dans lequel le procédé d'essai immunologique est l'immunochromatographie.

5. Procédé d'essai immunologique selon la revendication 4,

dans lequel l'étape de détection inclut ;
une étape de répartition consistant à répartir des corps composites de particules d'or sur un support insoluble ayant un site de réaction auquel une deuxième substance de liaison capable de se lier à un antigène dans la solution concentrée en antigène a été immobilisée, dans un état où les corps composites de particules d'or qui sont des corps composites de l'antigène et des particules d'or modifiées avec une première substance de liaison capable de se lier à l'antigène sont formés,
une étape de capture consistant à capturer les corps composites de particules d'or au site de réaction du support insoluble, et
une étape d'amplification par argent consistant à amplifier en argent le corps composite de particules d'or capturé dans l'étape de capture.

6. Procédé d'essai immunologique selon l'une quelconque des revendications 1 à 5, dans lequel dans l'étape de concentration, un gabarit de concentration (200, 300, 400) est utilisé, qui comprend :

un conteneur contenant le polymère superabsorbant,
dans lequel le conteneur a une partie d'incorporation configurée pour incorporer la solution contenant un antigène et une unité de décharge configurée pour décharger la solution concentrée en antigène.

7. Procédé d'essai immunologique selon la revendication 6, dans lequel le gabarit de concentration (200) comprend :

un cylindre (10) qui est le conteneur ; et
un piston (20) qui est insérable dans le cylindre.

8. Procédé d'essai immunologique selon la revendication 6, dans lequel le gabarit de concentration (300) comprend :

un tube intérieur (50) qui est le conteneur ; et
un tube extérieur (60) dans lequel le tube intérieur (50) est insérable et duquel le tube intérieur est amovible.

9. Procédé d'essai immunologique selon la revendication 6,

dans lequel le gabarit de concentration (400) a une forme de compte-gouttes ayant une partie de tube (70) et une partie de pompe (80), et
la partie de tube (70) est le conteneur.

# FIG. 1

DOWNSTREAM UPSTREAM

FIG. 2A

FIG. 2B

FIG. 2C

FIG. 2D

FIG. 3A    FIG. 3B    FIG. 3C    FIG. 3D

EP 4 040 151 B1

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5728453 B
- WO 2006007711 A1
- CN 109569023 A
- US 2013231460 A1
- US 2014087367 A1
- EP 1234165 A1
- WO 2017139153 A
- WO 2013129634 A
- US 6020117 A
- JP 2009150869 A
- JP 2015536375 A

### Non-patent literature cited in the description

- **XIE XING et al.** Nanofiltration'' Enabled by Super-Absorbent Polymer Beads for Concentrating Microorganisms in Water Samples. *SCIENTIFIC REPORTS*, 15 February 2016, vol. 6 (1)